(19) Europäisches Patentamt
European Patent Office
Office européen des brevets

(11) Veröffentlichungsnummer: **0 133 663**
**B1**

(12) # EUROPÄISCHE PATENTSCHRIFT

(45) Veröffentlichungstag der Patentschrift: **08.04.87**

(51) Int. Cl.⁴: **C 07 D 239/30**

(21) Anmeldenummer: **84108664.8**

(22) Anmeldetag: **23.07.84**

(54) **Neue Pyrimidine und ein Verfahren zur Herstellung von Pyrimidinen.**

(30) Priorität: **04.08.83 DE 3328154**

(43) Veröffentlichungstag der Anmeldung: **06.03.85 Patentblatt 85/10**

(45) Bekanntmachung des Hinweises auf die Patenterteilung: **08.04.87 Patentblatt 87/15**

(84) Benannte Vertragsstaaten: **BE CH DE FR GB IT LI**

(56) Entgegenhaltungen:
**EP-A-0 065 480**
**DE-A-1 931 640**
**GB-A-1 158 300**

**CHEMICAL ABSTRACTS, Band 65, Nr. 3, 1. August 1966, Columbus, Ohio, USA L.D. PROTSENKO" Synthesis of halogenated pyrimidines" Spalte 3869f**
**CHEMICAL ABSTRACTS, Band 98, Nr. 11, 14. März 1983, Columbus, Ohio, USA E. KLAUKE "Fluorinated heterocyclic compounds" Seite 555, Spalte 2, Zusammenfassung Nr. 89 312x**

(73) Patentinhaber: **BAYER AG, Konzernverwaltung RP Patentabteilung, D-5090 Leverkusen 1 Bayerwerk (DE)**

(72) Erfinder: **Klauke, Erich, Dr., Eichendorffweg 8, D-5068 Odenthal (DE)**
Erfinder: **Baasner, Bernd, Dr., Mozartstrasse 41, D-5090 Leverkusen (DE)**
Erfinder: **Schündehütte, Karl Heinz, Professor Dr., Klief 75, D-5090 Leverkusen 3 (DE)**

**EP 0 133 663 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft teilweise neue Pyrimidine und ein Verfahren zu ihrer Herstellung durch Umsetzung von fluorierten Pyrimidinen mit Chlorwasserstoff.

2,4,6-Trichlor-5-fluor-pyrimidin ist aus Ukr. chim. Ž. 32, (1966), Nr. 4, 378 bis 382 bekannt.

Es wurden neue Pyrimidine der Formel

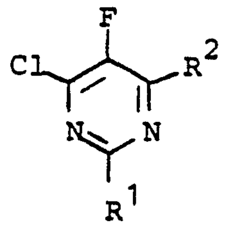

(I),

in der

$R^1$ Fluor oder Trifluormethyl und

$R^2$ Fluor, Chlor oder Trifluormethyl bedeutet, gefunden.

Die neuen Pyrimidine haben biozide, besonders fungizide Eigenschaften und dienen als Zwischenprodukte zur Herstellung cancerostatischer und viricider Verbindungen.

Es wurde auch ein Verfahren zur Herstellung von Pyrimidinen der Formel

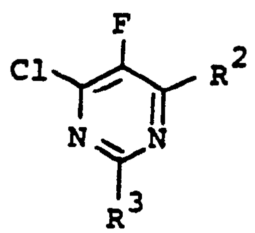

(II),

in der

$R^2$ und $R^3$ gleich oder verschieden sind und Fluor, Chlor oder Trifluormethyl bedeuten, gefunden, das dadurch gekennzeichnet ist, daß man Pyrimidine der Formel

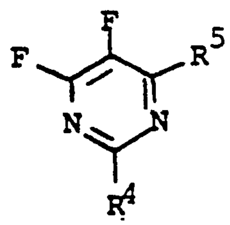

(III),

in der

$R^4$ und $R^5$ gleich oder verschieden sind und Fluor oder Trifluormethyl bedeuten, mit Chlorwasserstoff bei 3 bis 180 bar und bei Temperaturen im Bereich 120 bis 250° C umsetzt.

Nach dem Verfahren können ebenfalls die neuen Pyrimidine hergestellt werden.

Das erfindungsgemäße Verfahren kann anhand des folgenden Reaktionsschemas erläutert werden:

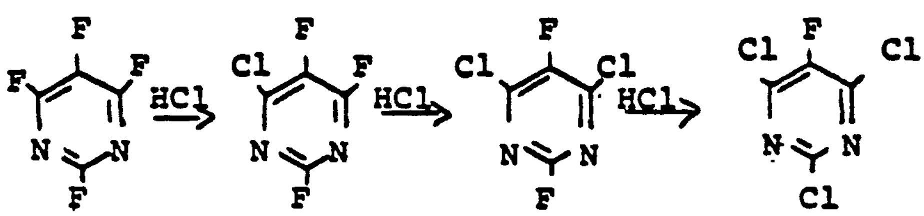


Die als Ausgangsverbindungen verwendeten Pyrimidine sind an sich bekannt (J. org. Chem. 27. (1962), 2580-2584, J. Chem. Soc. G, (1970), 1280). Sie können beispielsweise hergestellt werden, indem man die entsprechenden perchlorierten Verbindungen mit verschiedenen Fluor;erungsmitteln, gegebenenfalls stufenweise, durch Cl/F-Austausch fluoriert (J. Fluorine Chem. 21, (1982) S. 495).

Nach dem erfindungsgemäßen Verfahren erfolgt bevorzugt der stufenweise Austausch des Fluors im den

Ausgangspyrimidinen gegen Chlor durch Veränderung des Chlorwasserstoffdruckes.

So wird erfindungsgemäß der Fluorsubstituent in 6-Stellung des Pyrimidins bei einem Chlorwasserstoffdruck von 3 bis 50 bar gegen Chlor ausgetauscht. Bevorzugt erfolgt dieser Austausch bei einem Druck von 5 bis 30 bar.

Nachfolgend wird der Fluorsubstituent in 4-Stellung des Pyrimidins bei einem Chlorwasserstoffdruck von 12 bis 150 bar gegen Chlor ausgetauscht. Bevorzugt erfolgt dieser Austausch bei einem Chlorwasserstoffdruck von 15 bis 90 bar.

Nachfolgend wird der Fluorsubstituent in 2-Stellung des Pyrimidins bei einem Chlorwasserstoffdruck von 50 bis 180 bar gegen Chlor ausgetauscht. Bevorzugt erfolgt dieser Austausch bei einem Chlorwasserstoffdruck von 55 bis 120 bar.

Für den Fall, daß die 6-, 4- oder 2-Stellung nicht durch Fluor substituiert ist, entfällt selbstverständlich ein Austausch an dieser Stelle und der Austausch erfolgt in entsprechender Reihenfolge.

Das erfindungsgemäße Verfahren wird im Temperaturbereich von 120 bis 250°C, bevorzugt von 150 bis 200°C, durchgeführt.

Der Umsetzungsgrad Fluor gegen Chlor läßt sich durch die Parameter Druck und Temperatur sehr gut einstellen.

Das erfindungsgemäße Verfahren kann beispielsweise wie folgt durchgeführt werden:

In einem Autoklaven legt man die Ausgangsverbindung vor und stellt den gewünschten Chlorwasserstoffdruck ein. Es wird dann auf die erfindungsgemäße Reaktionstemperatur aufgeheizt, wobei das Einsetzen und der Verlauf der Umsetzung aim Abfall des HCl-Druckes beobachtet werden kann. Nach Ablauf der Umsetzung wird der Druck entspannt und vorzugsweise durch Destillation aufgearbeitet.

Die Durchführung des erfindungsgemäßen Verfahrens ist überraschend, weil im allgemeinen für die Fluor-Chlor-Austauschreaktion das Aluminiumchlorid das bevorzugte Reagenz ist (Houben-Weyl Bd. V/3, Seite 493 (1962)), und es nicht zu erwarten war, daß Chlorwasserstoff brauchbar ist.

Die erfindungsgemäßen Pyrimidine haben eine stark biozide Wirkung.

Die erfindungsgemäßen Pyrimidine können in Gegenwart eines Chlorwasserstoff-Akzeptors und eines Hydrierkatalysators zu in 4- oder 6-Stellung hydrierten Pyrimidinen umgesetzt werden.

Durch alkalische oder saure Verseifung erhält man die entsprechenden Pyrimidindione (Ureide) vom Typ des Wirkstoffs 5-Fluoruracil.

**Beispiel 1**

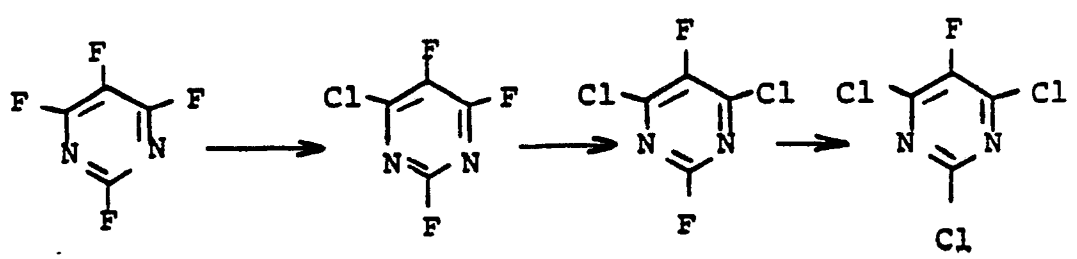

In einem VA-Autoklaven werden 200 g Tetrafluorpyrimidin vorgelegt und 30 bar Chlorwasserstoff gasförmig aufgedrückt und auf 160° hochgeheizt. Die bei der Reaktion verbrauchte Chlorwasserstoff-Menge wird laufend nachgedrückt bis der Druck konstant bleibt. Nach 4 Stunden wird abgekühlt, der überschüssige Chlorwasserstoff-Druck entspannt und der Ansatz durch Destillation aufgearbeitet. Man erhält 213 g Rohdestillat der gaschromatografischen Zusammensetzung 4 % Ausgangsmaterial, 16 % 2,4,5-Trifluor-6-chlorpyrimidin, 64 % 2,5-Difluor-4,6-dichlorpyrimidin, 14,5 % 5-Fluor-2,4,6-trichlor -pyrimidin. Durch fraktionierte Destillation werden die verschiedenen Pyrimidine isoliert und man erhält

I. 2,4,5-Trifluor-6-chlorpyrimidin als Flüssigkeit vom
Kp: 121°, $n_D^{20}$;1,4465

II. 2,5-Difluor-4,6-dichlorpyrimidin als Flüssigkeit
vom Kp: 162°, $n_D^{20}$: 1,5021

III. 5-Fluor-2,4,6-trichlorpyrimidin
Kp: 82°/14 mbar, Fp.: 37-8°

**Beispiel 2**

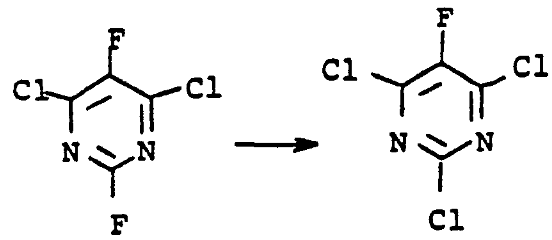

Im Autoklaven werden 433 g 2,5-Difluor-4,6-dichlor-pyrimidin vorgelegt und 3 h bei 200° mit 100 bar HCl behandelt. Nach Abkühlen und Entspannen erhält man 473 g eines kristallin flüssigen Gemisches der gaschromatografischen Zusammensetzung aus 38 % 2,5-Difluor-4,6-dichlor -pyrimidin und 60 % 2,4,6-Trichlor-5-fluorpyrimidin, das sich wie in Beispiel 1 beschrieben destillativ leicht trennen läßt.

**Beispeil 3**

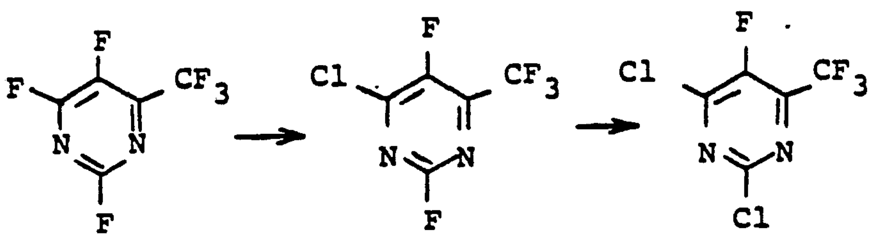

In einem Autoklaven werden 676 g 2,4,5-Trifluor-6-tri -fluormethylpyrimidin 4 Stunden bei einem HC1-Druck von 30 bar und einer Temperatur von 160°C gerührt. Nach Abkühlen und Entspannen erhält man 785 g Rohprodukt, aus dem durch Destillation 465 g 2,5-Difluor-4-chlor-6-trifluormethylpyrimidin als Flussigkeit vom KP.: 128°, $n_D^{20}$: 1.4132 und
62 g 5-Fluor-2,4-dichlor-6-trifluormethylpyrimidin
vom Kp.: 157-9°, $n_D^{20}$: 1.4520
isoliert werden.

**Beispiel 4**

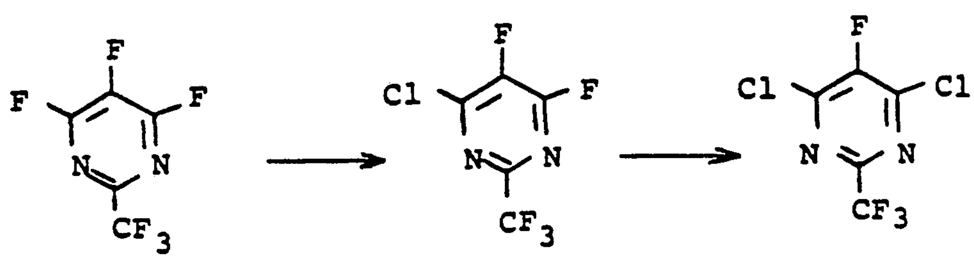

Wie in Beispiel 1 bis 3 beschrieben, lassen sich durch HCl-Behandlung unter Druck und erhöhter Temperatur aus 4,5,6-Trifluor-2-trifluormethylpyrimidin die Verbindungen 2-Trifluormethyl-4,5-difluor-6-chlorpyrimidin Kp: 127°, $n_D^{20}$: 1.4091 und 2-Trifluormethyl-4,6-dichlor-5-fluorpyrimidin Kp. 159°, $n_D^{20}$: 1.4520 herstellen.

**Beispiel 5**

Herstellung von

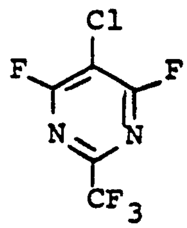

und

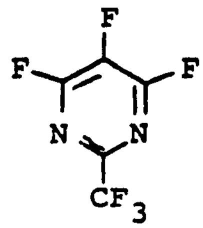

In einem 500 ml Dreihalskolben werden 200 g 4,6-Difluor-5-chlor-2-trichlormethylpyrimidin vorgelegt und nach Zugabe von 135 g $Sb_3F$ und 10 ml $SbCl_5$ langsam hochgeheizt und 45 Minuten bei der Rückflußtemperatur von 134° gerührt. Der Ansatz wird abgekühlt, mit verdünnter HCl gewaschen der organische Anteil mit 300 ml $CH_2Cl_2$ aufgenommen und über $MgSO_4$ getrocknet. Durch Destillation gewinnt man daraus 119 g 2,4-Difluor-5-chlor-2-trifluormethylpyrimidin von Kp. 127°, $n_D^{20}$: 1.4095.

In einem Druckgefäß aus Edelstahl werden 534 g der obigen Verbindung mit 310 g KF (wasserfrei) in 640 ml Tetramethylensulfon Vorgelegt und ein Stickstoffschutzdruck von 3 bar aufgegeben. Dann heizt man unter kräftigem Rühren bis auf 200° und läßt 4 Stunden bei dieser Temperatur reagieren. Nach dem Abkühlen wird aus einer Glasapparatur destilliert.

Ausbeute: 449 g Perfluor-(2-methylpyrimidin)

Kp.: 98°, $n_D^{20}$: 1.3662.

**Patentansprüche**

1. Pyrimidine der Formel

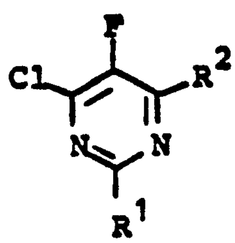

,

in der

$R^1$ Fluor oder Trifluormethyl und

$R^2$ Fluor, Chlor oder Trifluormethyl bedeuten.

2. Verfahren zur Herstellung von Pyrimidinen der Formel

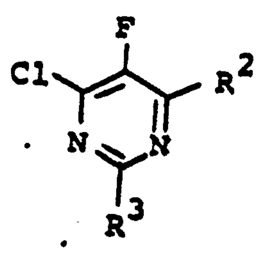

,

in der

$R^2$ und $R^3$ gleich oder verschieden sind und Fluor, Chlor oder Trifluormethyl bedeuten, dadurch gekennzeichnet, daß man Pyrimidine der Formel

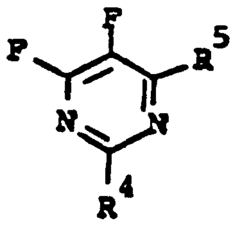

in der

$R^4$ und $R^5$ gleich oder verschieden sind und Fluor oder Trifluormethyl bedeuten,

mit Chlorwasserstoff bei 3 bis 180 bar und bei Temperaturen im Bereich 120 bis 250°C umsetzt.

3. Verfahren nach Anspruch 2, dadurch gekennzeichnet, daß man einen Fluorsubstituenten in 6-Stellung des Pyrimidins bei einem Chlorwasserstoffdruck von 3 bis 50 bar gegen Chlor austauscht.

4. Verfahren nach den Ansprüchen 2 und 3, dadurch gekennzeichnet, daß man nachfolgend einen Fluorsubstituenten in 4-Stellung des Pyrimidins bei einem Chlorwasserstoffdruck von 12 bis 150 bar gegen Chlor austauscht.

5. Verfahren nach den Ansprüchen 2 bis 4, dadurch gekennzeichnet, daß man nachfolgend einen Fluorsubstituenten in 2-Stellung des Pyrimidins bei einen Chlorwasserstoffdruck von 50 bis 180 bar gegen Chlor austauscht.

## Claims

1. Pyrimidines of the formula

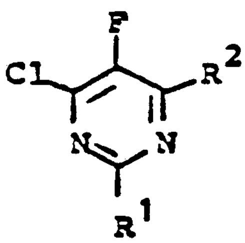

in which

$R^1$ denotes fluorine or trifluoromethyl, and

$R^2$ denotes fluorine, chlorine or trifluoromethyl.

2. Process for the preparation of pyrimidines of the formula

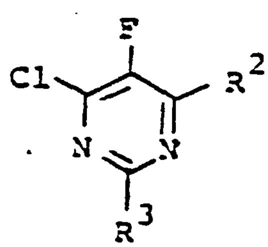

in which

$R^2$ and $R^3$ are identical or different and denote fluorine chlorine or trifluoromethyl, characterised in that pyrimidines of the formula

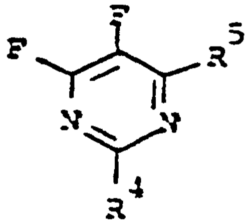

in which $R^4$ and $R^5$ are identical or different and denote fluorine or trifluoromethyl, are reacted with hydrogen chloride at 3 to 180 bar and at temperatures in the range 120 to 250° C.

3. Process according to Claim 2, characterised in that a fluorine substituent in the 6-position of the pyrimidine is replaced by chlorine under a pressure of hydrogen chloride of 3 to 50 bar.

4. Process according to Claims 2 and 3, characterised in that subsequently a fluorine substituent in the 4-position of the pyrimidine is replaced by chlorine under a pressure of hydrogen chloride of 12 to 150 bar.

5. Process according to Caims 2 to 4, characterised in that subsequently a fluorine substituent in the 2-position of the pyrimidine is replaced by chlorine under a pressure of hydrogen chloride of 50 to 180 bar.

**Revendications**

1. Pyrimidines de formule:

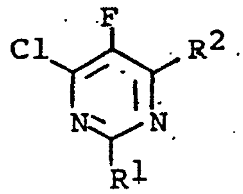

dans laquelle
$R^1$ signifie du fluor ou trifluorométhyle et
$R^2$ du fluor, du chlore ou trifluorométhyle.

2. Procédé de fabrication de pyrimidines de formule:

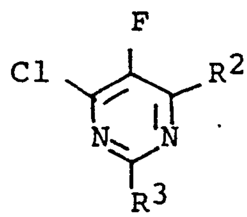

dans laquelle
$R^2$ et $R^3$ sont identiques ou différents et signifient du fluor, du chlore ou trifluorométhyle, caractérisé en ce qu'on fait réagir des pyrimidines de formule

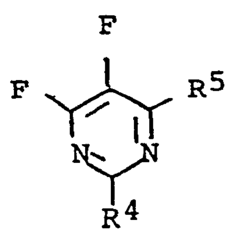

dans laquelle
$R^4$ et $R^5$ sont identiques ou différents et signifient du fluor ou trifluorométhyle, avec de l'acide chlorhydrique sous 3 à 180 bars et à des températures dans l'intervalle de 120 à 250° C

3. Procédé selon la revendication 2, caractérisé en ce qu'on échange un substituent fluor en position 6 de la pyrimidine sous une pression d'acide chlorhydrique de 3 à 50 bars contre du chlore.

4. Procédé selon les revendications 2 et 3, caractérisé en ce que par la suite on échange un substituant fluor en position 4 de la pyrimidine sous une pression d'acide chlorhydrique de 12 à 150 bars contre du chlore.

5. Procédé selon les revendications 2 à 4, caractérisé en ce que par la suite on échange un substituant fluor en position 2 de la pyrimidine sous une pression d'acide chlorhydrique de 50 à 180 bars contre du chlore.